# EUROPEAN PATENT APPLICATION

(11) **EP 4 591 844 A1**
(43) Date of publication of application: **30.07.2025**
(21) Application number: 24218282.2
(22) Date of filing: 09.12.2024
(51) Int. Cl.: A61F 13/551

(54) **A CONSUMER PRODUCT COMPRISING AT LEAST ONE ABSORBENT SANITARY ARTICLE AND AN ENVELOPE OF PAPER MATERIAL**

(30) Priority: 24.01.2024 IT 202400001323
(71) Applicant: Fameccanica.Data S.p.A., 66020 San Giovanni Teatino (CH) (IT)
(72) Inventor: MANCINI, Osvaldo, I-66020 San Giovanni Teatino (Chieti) (IT); BONELLI, Guido, I-66020 San Giovanni Teatino (Chieti) (IT)
(74) Representative: Buzzi, Notaro & Antonielli d'Oulx S.p.A.

(57) **Abstract**

A consumer product comprising at least one absorbent sanitary article (12) enclosed in an envelope (14), wherein the envelope (14) comprises at least one sheet of paper material (16) having a basis weight between 20-80 g/m², wherein at least one perimeter portion of the sheet of paper material (16) is fixed to another perimeter portion of the same sheet of paper material (16) or of another sheet of paper material (16) by means of an embossing seam (30) having a width (L) between 4-20 mm.

## Description

### Field of the invention

The present invention relates in general to the packaging of absorbent sanitary articles, in particular flat absorbent sanitary articles, having a thickness lower than the other dimensions, and folded absorbent sanitary articles whose resulting thickness is lower than the other dimensions of the folded article.

In particular, the invention relates to a consumer product comprising at least one absorbent sanitary article and an envelope of paper material.

The invention was developed in particular with a view to the packaging of feminine hygiene products such as pantyliners, light inco, panty shields, or the like.

In the following description, reference will be made to this specific field without however losing generality.

### Description of the prior art

Absorbent sanitary products with thin shapes, such as feminine hygiene products, are often individually packaged in their own envelopes commonly called envelopes or pouches.

The individual packaging of absorbent sanitary articles may be carried out by enclosing the individual absorbent sanitary articles between two flexible sheets superimposed on each other which enclose a respective absorbent sanitary article in the manner of a sandwich and joined together at their edges by means of adhesive.

Solutions are also known wherein each absorbent sanitary article is closed in a casing made up of a single sheet of flexible material folded to form two or more flaps joined together at their edges by means of adhesive.

The packages may be made up of sheets of plastic material, for example polyethylene, or paper material.

Manufacturers and users of packaging materials are increasingly using paper materials as a replacement for plastic materials to reduce environmental impact.

However, in the field of packaging of sanitary articles, the environmental sustainability benefits resulting from the use of paper material instead of plastic sheets are partially nullified by the fact that paper packages are closed using synthetic adhesives that compromise the biodegradability of the areas on which the adhesive is applied.

From the document IT202022000000080 by the same applicant, a consumer product is known comprising an absorbent sanitary article enclosed in a paper envelope closed with a biodegradable adhesive. Using a biodegradable adhesive maintains the biodegradability of the packaging. However, the solution known from this document requires applying strips of adhesive along the edges of the sheets of paper material, and this causes several problems.

### Object and summary of the invention

The present invention aims to provide a consumer product comprising at least one absorbent sanitary article and a paper envelope, which overcome the problems of known solutions.

According to the present invention, this object is achieved by a consumer product having the characteristics forming the subject of claim 1.

Preferred embodiments form the subject of the dependent claims.

### Brief description of the drawings

The present invention will now be described in detail with reference to the attached drawings, given purely by way of non-limiting example, wherein:
- Figures 1, 2 and 3 are perspective views illustrating respectively a first, a second and a third embodiment of a consumer product according to the present invention in an open configuration,
- Figures 4, 5 and 6 are plan views illustrating the consumer products of Figures 1, 2 and 3 in a closed configuration,
- Figures 7, 8 and 9 are plan views illustrating alternative embodiments of the consumer products of Figures 4, 5 and 6,
- Figures 10-15 are enlarged details illustrating possible embossing patterns of consumer products according to the present invention,
- Figure 16 is an enlarged schematic cross-section illustrating the protrusions between embossing elements, and
- Figures 17, 18, 19 and 20 are plan views illustrating possible embodiments of consumer products according to the present invention.

### Detailed description

With reference to the figures, numeral 10 indicates a consumer product according to the present invention. The consumer product 10 comprises at least one absorbent sanitary article 12 enclosed in an envelope 14. The envelope 14 includes at least one sheet of paper material 16.

The absorbent sanitary articles 12 may be feminine hygiene products such as pantyliners, light inco, panty liners, or the like. In the following description, reference will be made to the case wherein each consumer product 10 comprises only one absorbent sanitary article 12 but it is understood that each consumer product 10 may comprise two or more absorbent sanitary articles 12 enclosed in the same envelope 14.

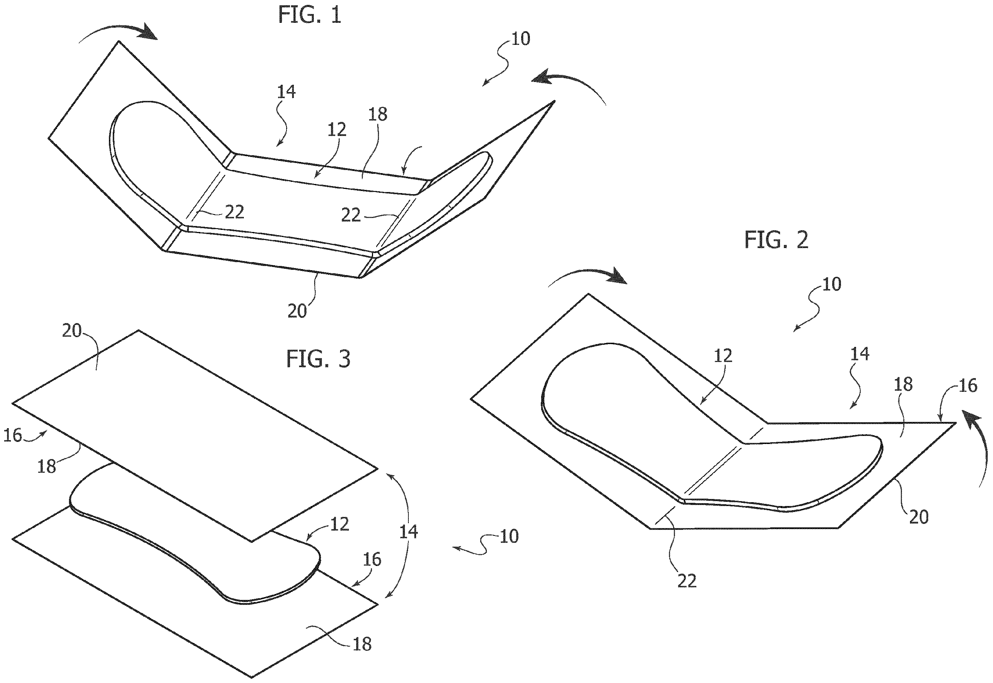

With reference to figure 1, the envelope 14 may be formed from a single sheet of paper material 16 having an inner surface 18 and an outer surface 20. The sanitary article 12 is applied to the inner surface 18 of the paper material sheet 16. The sheet of paper material 16 and the absorbent sanitary article 12 are folded along two folding lines 22 parallel to each other so as to form a tri-folded envelope 14 which contains a tri-folded absorbent sanitary article 12 inside it.

In the embodiment illustrated in Figure 2, the sheet of paper material 16 and the absorbent sanitary article 12 are folded along a single folding line 22 so as to form a double-folded envelope 14 which encloses a double-folded absorbent sanitary article 12 inside it.

In the embodiment illustrated in Figure 3, the envelope 14 comprises two sheets of paper material 16 having respective inner surfaces 18 facing each other. The two sheets of paper material 16 enclose a respective absorbent sanitary article 12 between them in a sandwich-like fashion. The absorbent sanitary article 12 may be extended, i.e. not folded.

Said at least one sheet of paper material 16 may be made of biodegradable kraft paper. The paper material sheet 16 may be compostable as well as biodegradable. The sheet of paper material 16 has a basis weight between 20-80 g/m².

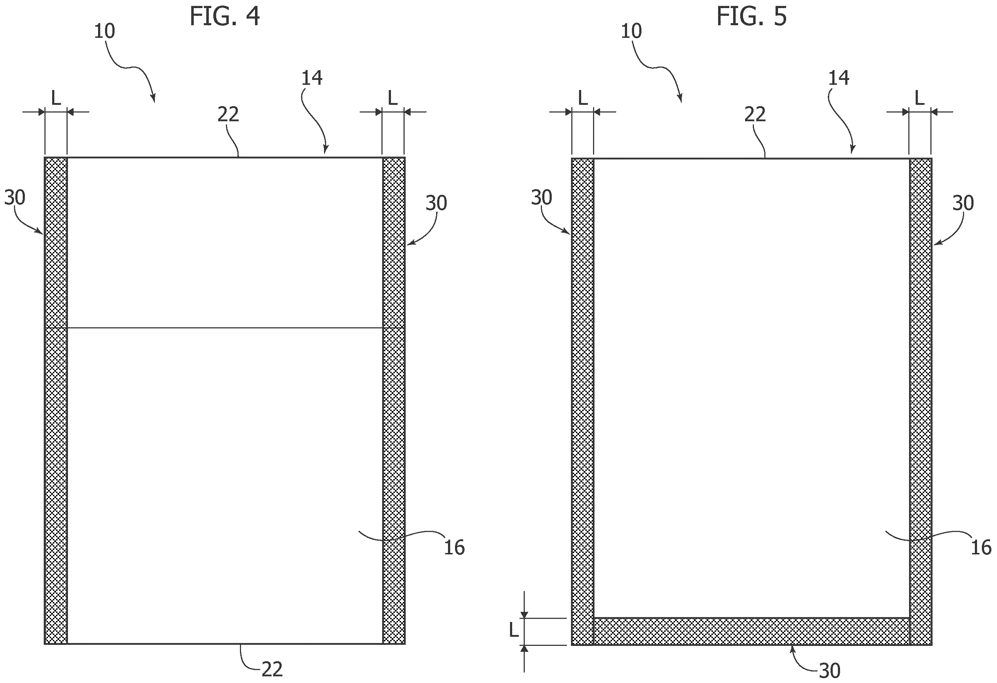

With reference to Figures 4-9, at least one perimeter portion of the sheet of paper material 16 is fixed to another perimeter portion of the same sheet of paper material 16 or of another sheet of paper material 16 by means of an embossing seam 30.

Embossing is a mechanical and/or thermal process that consists of applying pressure and/or heat to a surface in order to give it a permanent three-dimensional relief. This technique is often used to create decorative patterns, raised text or other tactile features on materials such as paper, cardboard, plastics or other substrates.

On a sheet of paper material, embossing produces a permanent deformation of the cellulose fibres which generates complementary protrusions and recesses on opposite surfaces of the sheet of paper material.

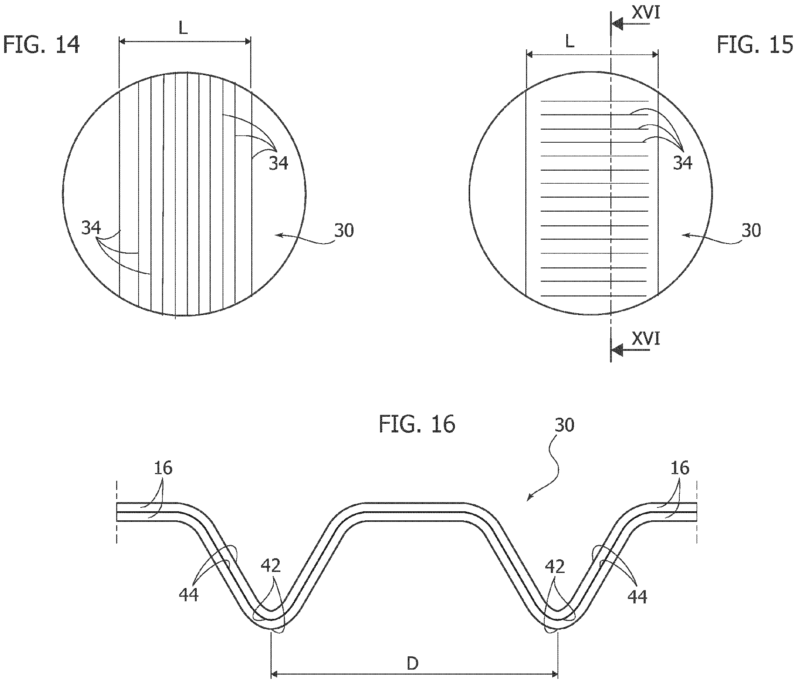

With reference to Figure 16, the embossing of two layers of paper material superimposed on each other produces protrusions 42 formed on a portion of the sheet of paper material 16 engaging complementary recesses 44 formed on another portion of the same sheet of paper material 16 or of another sheet of paper material 16.

In cross-section, the protrusions 42 and the recesses 44 may have a generally conical shape.

The engagement between the protrusions 42 and the recesses 44 produces a mechanical joint between the overlapping portions of paper material.

With reference to Figure 16, the distance between adjacent protrusions 42 is indicated by D. The average distance between adjacent protrusions 42, calculated as the arithmetic mean between the distances D, is between 0.5 mm and 4.5 mm.

Referring to Figures 4-9, each embossing seam 30 has a width L between 4-20 mm.

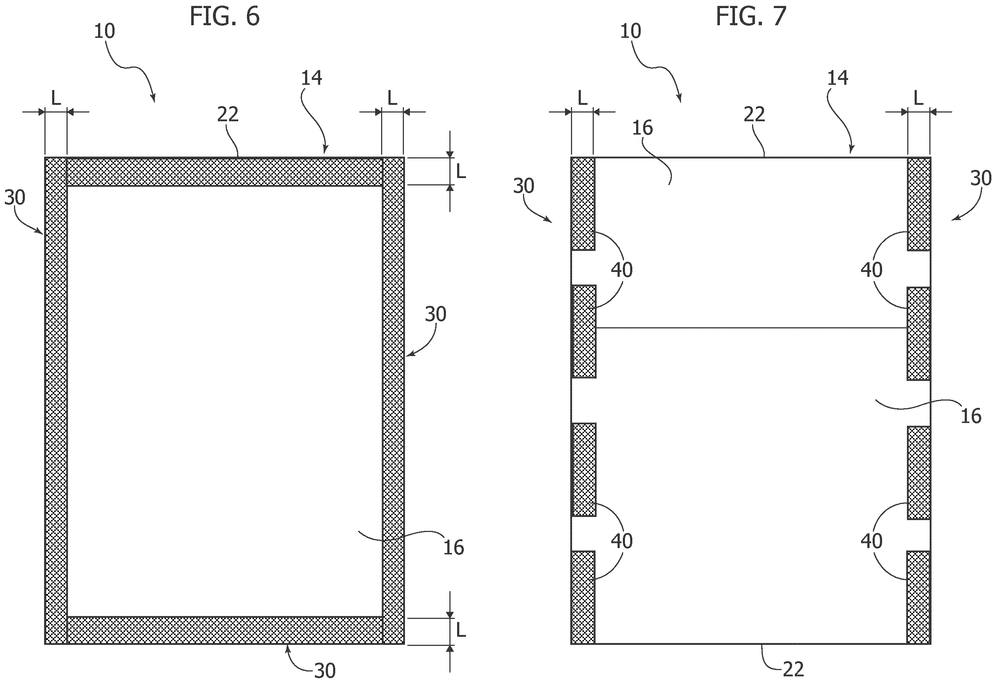

In the embodiments of Figures 4 and 7, the envelope 14 is triple-folded and is closed by means of two embossing seams 30 extending along respective sides of the envelope 14 orthogonal to the folding lines 22.

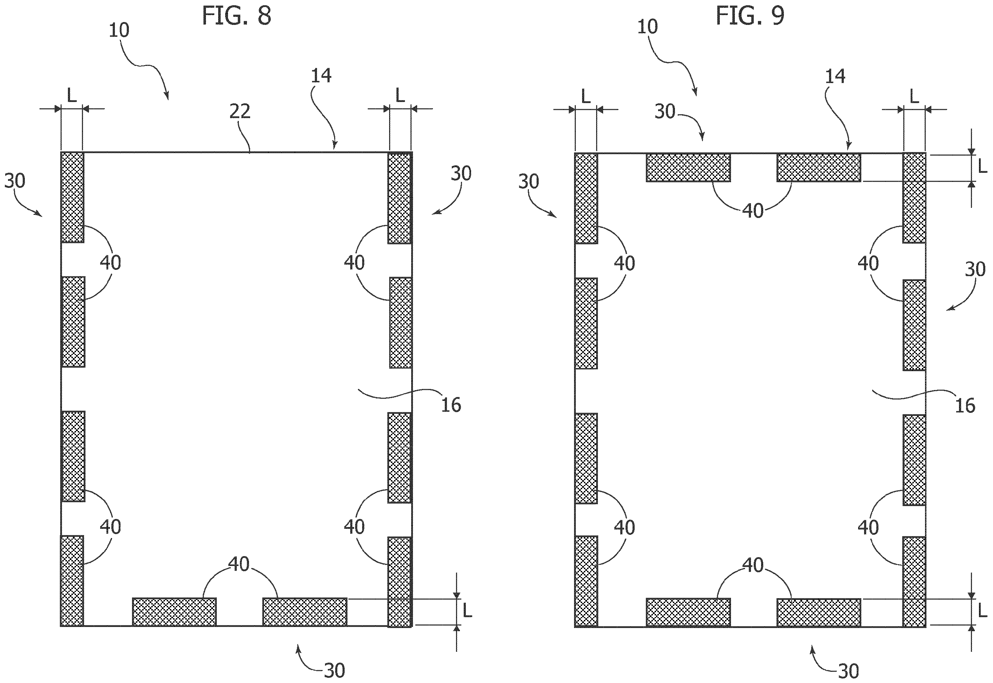

In the embodiments of Figures 5 and 8, the envelope 14 is double-folded and closed by three embossing cords 30, two of which extend along respective sides of the envelope 14 orthogonal to the folding lines 22 and one of which extends along the side of the envelope 14 opposite the folding line 22.

In the embodiments of Figures 6 and 9, the envelope 14 is formed by two sheets of paper material 16 which are not folded and superimposed on each other and is closed by four embossing seams 30 extending along respective sides of the envelope 14.

In the embodiments illustrated in Figures 4-6 each embossing seam 30 extends continuously along a respective side of the envelope 14.

In the embodiments illustrated in Figures 7-9 each embossing seam 30 is intermittent and comprises a plurality of embossing areas 40 spaced apart along a respective side of the envelope 14.

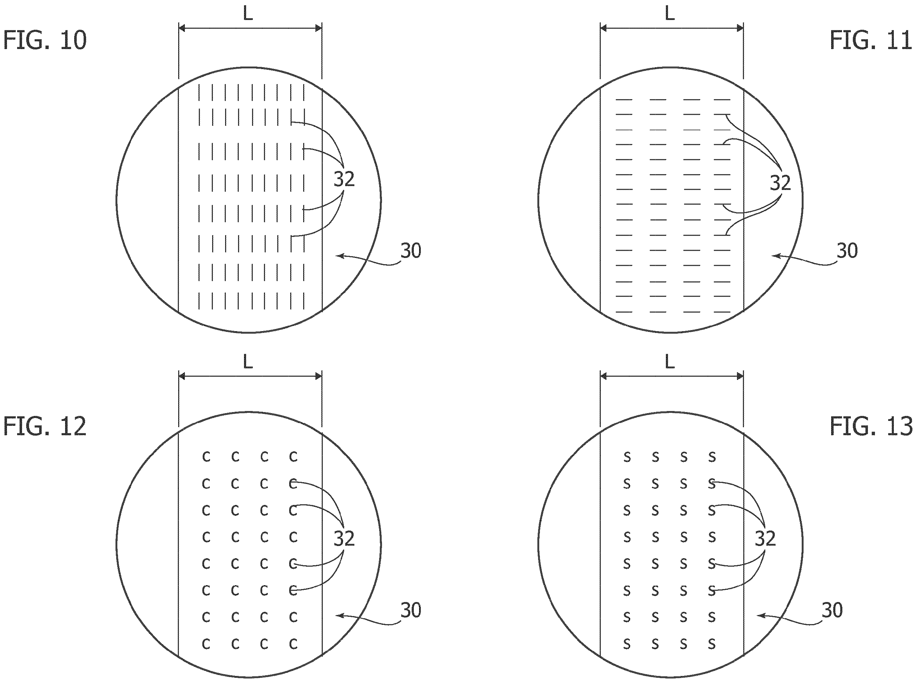

With reference to Figures 10-13, in one possible embodiment the protrusions 42 may be formed by individual embossing elements 32.

The individual embossing elements 32 are distributed according to an embossing pattern having a variable density depending on the size of the embossing elements, for example, in the order of 10 elements/cm². The individual embossing elements 32 may be formed of straight segments, for example, with an I-shape or dashed as illustrated in Figures 10 and 11. The embossing pattern could be formed by individual embossing elements 32 formed by curved lines, for example, with a C- or S-shape as illustrated in Figures 12 and 13.

With reference to Figures 14 and 15, in a possible embodiment the protrusions 42 may be formed by embossing lines 34 parallel to each other. The embossing lines 34 may extend parallel or transversely to the respective side of the envelope 14.

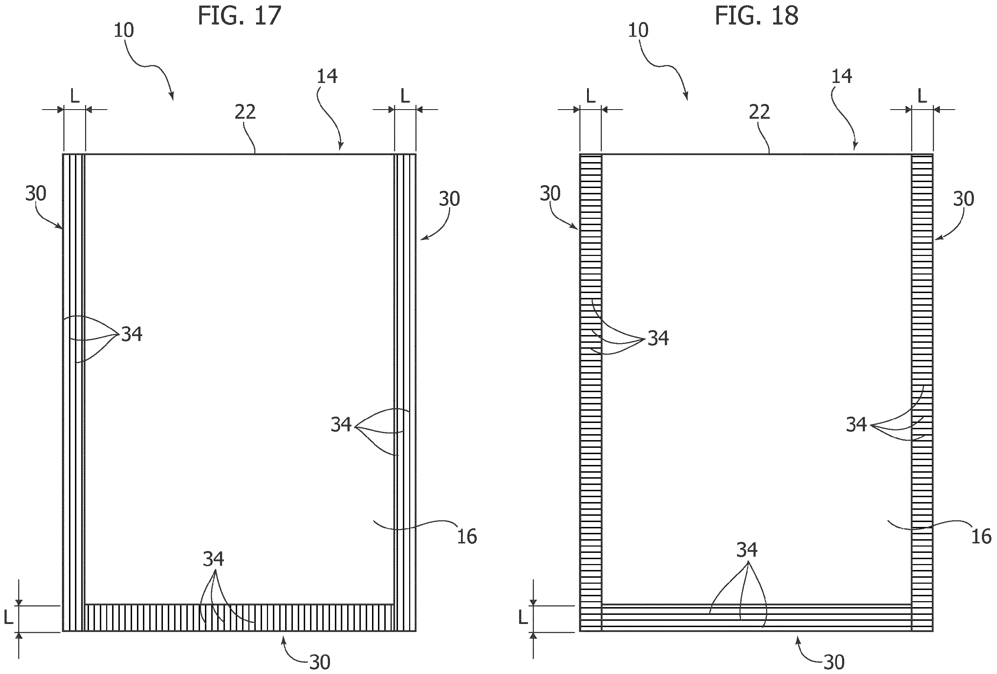

Figures 17-20 illustrate possible embodiments of line embossing patterns. Figures 17-20 illustrate as an example the case of a double-folded envelope 14, but it is understood that similar embossing patterns may be used in the case of a tri-folded or unfolded envelope.

In the embodiment illustrated in Figure 17, the embossing lines 34 are parallel to the respective side of the envelope 14 in the embossing seams 30 orthogonal to the folding line 22 and transverse to the respective side of the envelope 14 in the embossing seam 30 parallel to the folding line 22.

In the embodiment illustrated in Figure 18, the embossing lines 34 are transverse to the respective side of the envelope 14 in the embossing seams 30 orthogonal to the folding line 22 and parallel to the respective side of the envelope 14 in the embossing seam 30 parallel to the folding line 22.

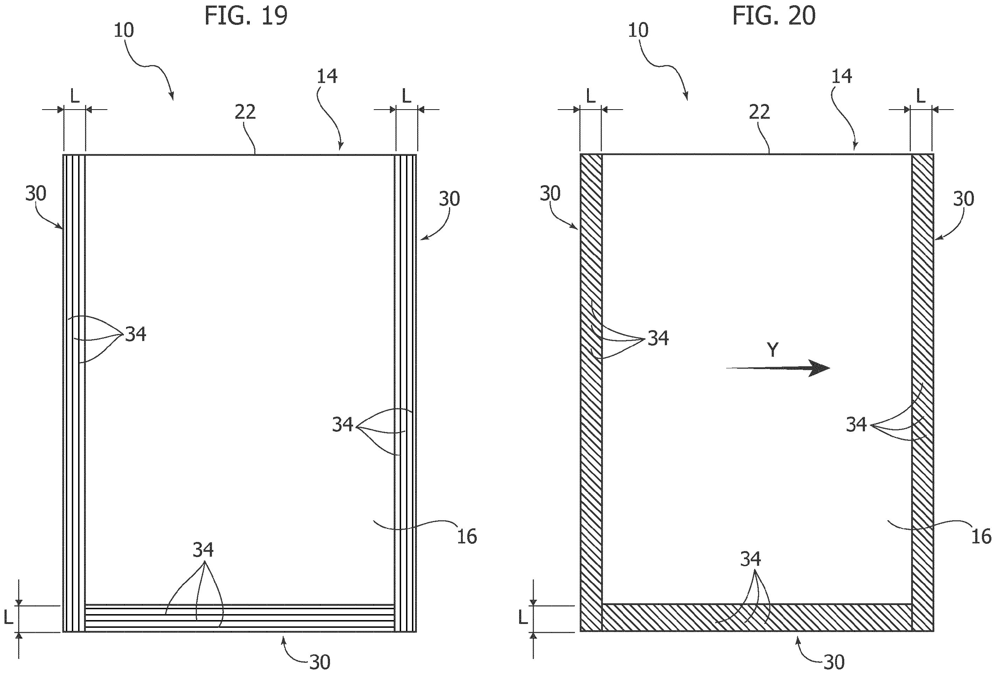

In the embodiment illustrated in Figure 19, the embossing lines 34 are parallel to the respective side of the envelope 14 both in the embossing seams 30 orthogonal to the folding line 22 and in the embossing seam 30 parallel to the folding line 22.

In the embodiment illustrated in Figure 20, the embossing lines 34 are inclined, for example by 45°, with respect to the respective side of the envelope 14 both in the embossing seams 30 orthogonal to the folding line 22 and in the embossing seam 30 parallel to the folding line 22.

In possible embodiments, the sheet of paper material 16 may be made exclusively of cellulose fibres. In this case, the mutual junction between the side portions of the sheet (or sheets) of paper material 16 along the embossing seams 30 is entrusted solely to the mechanical action of mutual engagement of the protrusions and recesses 42, 44. To facilitate the mutual adhesion between the protrusions and recesses 42, 44, the sheet of paper material 16 may be supplemented with a quantity of water between 10-20 gsm.

In the event wherein the mechanical action of mutual engagement of the protrusions and recesses 42, 44 alone is not sufficient to ensure the fixing between the side portions of the sheet (or sheets) of paper material 16, the sheet of paper material 16 (or at least one of the sheets of paper material 16) may comprise adhesive materials.

The adhesive materials may be biodegradable adhesive materials of natural origin such as beeswax, natural rubber (caoutchouc), pine resin and similar plant resins, plasticizers based on vegetable or animal oil. The adhesive materials may also be of synthetic origin, for example, PVA, PE, alkyd resins, etc.

In possible embodiments the adhesive materials may be dispersed between the cellulose fibres of the paper material sheet 16. The amount of adhesive materials dispersed between the fibres may be less than 20% by weight.

In possible embodiments the adhesive materials may be provided in the form of a film applied to the inner surface 18 of the sheet of paper material 16 or at least one of the sheets of paper material 16. The amount of adhesive materials provided in film form may be less than 10% by weight.

The absorbent sanitary article 12 may be held on the inner surface 18 of the paper material sheet 16 by a layer of positioning adhesive. The inner surface 18 of the paper sheet 16 on which the absorbent sanitary article 12 is applied may be siliconed to allow the positioning adhesive to be removed. The silicone layer may extend over the entire inner surface 18 of the sheet of paper material 16 or may extend only in the central area of the inner surface 18, so as to leave the edges on which the embossing seams are formed without a silicone layer.

Of course, without prejudice to the principle of the invention, the details of construction and the embodiments can be widely varied with respect to those described and illustrated, without thereby departing from the scope of the invention as defined by the claims that follow.

## Claims

1. A consumer product comprising at least one absorbent sanitary article (12) enclosed in an envelope (14), wherein said envelope (14) includes at least one sheet of paper material (16) having a basis weight between 20-80 g/m², wherein at least one peripheral portion of said sheet of paper material (16) is fixed to another peripheral portion of the same sheet of paper material (16) or to another sheet of paper material (16) by means of an embossing seam (30) having a width (L) between 4-20 mm, wherein said embossing seam (30) includes a plurality of projections (42) and recesses (44) engaging each other and wherein an average distance between adjacent projections (42), calculated as the arithmetic mean of the distances (D) between adjacent projections (42), is comprised between 0.5 mm and 4.5 mm.

2. A consumer product according to claim 1, wherein said projections (42) and recesses (44) are formed by individual embossing elements (32).

3. A consumer product according to claim 2, wherein said projections (42) and recesses (44) are formed by embossing lines (34) parallel to each other.

4. A consumer product according to any of the preceding claims, wherein said embossing seam (30) extends continuously along a respective side of the pouch (14) .

5. A consumer product according to any of claims 1-3, wherein said embossing seam (30) is intermittent and includes a plurality of embossing areas (40) spaced apart along a respective side of the envelope (14).

6. A consumer product according to any of the preceding claims, wherein the sheet of paper material (16) is supplemented with a quantity of water comprised between 10-20 gsm.

7. A consumer product according to any of claims 1-5, wherein said sheet of paper material (16) comprises adhesive materials.

8. A consumer product according to claim 7, wherein said adhesive materials are dispersed among cellulose fibers of the sheet of paper material (16) in an amount lower than 20% by weight.

9. A consumer product according to claim 7, wherein said adhesive materials are provided in the form of a film applied to an inner surface (18) of at least one sheet of paper material (16) in an amount less than 10% by weight.

10. A consumer product according to any of the preceding claims, wherein said envelope (14) comprises:
- a single sheet of paper material (16) folded around two folding lines (22) parallel to each other,
- a single sheet of paper material (16) folded around a single folding line (22), or
- two sheets of paper material (16) overlapping each other.
